# EUROPEAN PATENT APPLICATION

(11) **EP 4 835 443 A2**
(43) Date of publication of application: **04.11.2026**
(21) Application number: 26200871.7
(22) Date of filing: 14.04.2023
(51) Int. Cl.: G01N 15/14

(54) **SAMPLE ANALYZER AND PLATELET COUNTING METHOD**

(30) Priority: 14.04.2022 CN 202210388780
(62) Document number(s) of the earlier application(s) in accordance with Art. 76 EPC: 23167888.9 / 4 261 524
(71) Applicant: Shenzhen Mindray Bio-Medical Electronics Co., Ltd., Shenzhen, Guangdong 518057 (CN)
(72) Inventor: ZHENG, Wenbo, Shenzhen, 518057 (CN); YE, Bo, Shenzhen, 518057 (CN); YAO, Donglan, Shenzhen, 518057 (CN); QI, Huan, Shenzhen, 518057 (CN); YE, Yi, Shenzhen, 518057 (CN)
(74) Representative: KIPA AB

(57) **Abstract**

The disclosure provides a sample analyzer and a platelet counting method. The sample analyzer includes a sample preparation device, an impedance testing device, an optical testing device, and a controller.

## Description

### TECHNICAL FIELD

The disclosure relates to the field of in vitro diagnostics, in particular to a sample analyzer and a platelet counting method.

### BACKGROUND

Platelet count is an important test item for clinical diagnosis and treatment of platelet (PLT) decrease-related diseases caused by various reasons. When the platelet count of a patient is lower than 20 × 10^{^9}/L, it is conventionally believed that platelet transfusion must be given to the patient, otherwise, the patient will be at risk of life-threatening bleeding. A platelet count threshold of 20 × 10^{^9}/L is a medical decision level for prophylactic platelet transfusion in clinical practices.

In recent years, there has been a trend to reduce prophylactic platelet transfusion in clinical practices, because the risk of random bleeding has not increased significantly after the threshold is lowered from 20 × 10^{^9}/L to 10 × 10^{^9}/L. Studies have shown that if clinicians have sufficient confidence in the reliability of platelet counts for evaluating the risk of bleeding, the medical decision level for prophylactic platelet transfusion can be lowered to 5 × 10^{^9}/L. In order to ensure the correctness and safety of clinical diagnosis, the platelet count should be both precise and accurate. Thus, accurate counting of platelets has important clinical significance.

For existing blood cell counting instruments, an impedance method is generally used for platelet counting. However, interferences from abnormal blood samples may lead to inaccurate results of platelet counting based on the impedance method, resulting in a clinical diagnosis error. For this reason, flow cytometry is used in some blood cell counting instruments for platelet counting, which eliminates the interferences mentioned above. However, for this purpose, it is necessary to additionally provide a special optical platelet testing channel for counting platelets, which not only increases the cost of clinical tests, but also leads to a significant increase in the volume and complexity of the instruments.

Thus, there is a need to obtain accurate platelet counting results at a cost as low as possible to assist in clinical diagnosis.

### SUMMARY

Thus, the object of the disclosure is to provide a sample analyzer and a corresponding platelet counting method, which can improve the accuracy of platelet counting without increasing the detection cost as much as possible.

In order to achieve the above object of the disclosure, a first aspect of the disclosure firstly provides a sample analyzer, including:
a sample preparation device configured to prepare a first test sample containing a first part of a test blood sample and a diluent, and to prepare a second test sample containing a second part of the test blood sample, a hemolysis reagent for hemolyzing red blood cells and a first stain reagent;
an impedance testing device including a first flow chamber and a detection component, the first flow chamber being configured to allow the first test sample to pass through, and the detection component being configured to obtain electronic information as the first test sample passes through the first flow chamber;
an optical testing device including a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light when it passes through the second flow chamber; and
a controller configured to:
   control the impedance testing device to test the first test sample, so as to obtain a first platelet counting result for the test blood sample based on the electronic information of the first test sample; control the optical testing device to test the second test sample, so as to obtain a second platelet counting result for the test blood sample based only on the optical information of the second test sample or so as to obtain a second platelet counting result for the test blood sample based on both the electronic information of the first test sample and the optical information of the second test sample; determining whether the first platelet counting result is unreliable due to a first abnormality of the test blood sample; and output the first platelet counting result and/or the second platelet counting result according to the result of the determination.

In the sample analyzer provided in the first aspect of the disclosure, two platelet counting results, namely, the first platelet counting result and the second platelet counting result, are obtained at a low cost through a testing channel based on impedance method and a hemolysis optical testing channel, and the first platelet counting result and/or the second platelet counting result are/is selectively outputted according to the result of determining whether the first platelet counting result is unreliable due to the abnormality of the test blood sample, thereby ensuring the accuracy of platelet counting results for abnormal samples.

A second aspect of the disclosure provides another sample analyzer, including:
a sample preparation device configured to prepare a first test sample containing a first part of a test blood sample and a diluent, and to prepare a second test sample containing a second part of the test blood sample, a hemolysis reagent for hemolyzing red blood cells and a first stain reagent;
an impedance testing device including a first flow chamber and a detection component, the first flow chamber being configured to allow the first test sample to pass through, and the detection component being configured to obtain electronic information as the first test sample passes through the first flow chamber;
an optical testing device including a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light when it passes through the second flow chamber; and
a controller configured to:
   control the impedance testing device to test the first test sample, so as to obtain the electronic information of the first test sample; control the optical testing device to test the second test sample, so as to obtain the optical information of the second test sample; obtaining a second platelet counting result for the test blood sample based on the electronic information of the first test sample and the optical information of the second test sample; and control, when the second platelet counting result is unreliable due to an abnormality of the test blood sample, the sample preparation device to prepare a third test sample containing a third part of the test blood sample, a diluent and a second stain reagent, and control the optical testing device to test the third test sample so as to obtain and output a third platelet counting result based on optical information of the third test sample.

In the sample analyzer provided in the second aspect of the disclosure, accurate platelet counting results can be obtained through a testing channel based on impedance method combined with an existing hemolysis optical testing channel, and an additional dedicated optical testing channel is used for platelet retest only when the platelet counting result is unreliable, so that the accuracy of platelet counts for abnormal samples can be improved with little overall increase in test cost.

A third aspect of the disclosure provides a corresponding platelet counting method, including:
preparing a first test sample containing a first part of a test blood sample and a diluent, and preparing a second test sample containing a second part of the test blood sample, a hemolysis reagent for hemolyzing red blood cells and a first stain reagent;
allowing the first test sample to pass through a first flow chamber, and detecting electronic information as the first test sample passes through the first flow chamber;
allowing the second test sample to pass through a second flow chamber, irradiating the second test sample as the second test sample passes through the second flow chamber with a light, and detecting optical information generated by the second test sample after being irradiated with the light;
obtaining a first platelet counting result for the test blood sample based on the electronic information of the first test sample;
obtaining a second platelet counting result for the test blood sample based only on the optical information of the second test sample, or obtaining a second platelet counting result for the test blood sample based on both the electronic information of the first test sample and the optical information of the second test sample;
determining whether the first platelet counting result is unreliable due to a first abnormality of the test blood sample; and
outputting the first platelet counting result and/or the second platelet counting result based on the result of the determination.

The platelet counting method according to the third aspect of the disclosure is particularly applicable to the sample analyzer according to the first aspect of the disclosure.

For the features and advantages of the sample analysis method according to the third aspect of the disclosure, reference can be made to the foregoing description of the sample analyzer according to the first aspect of the disclosure.

A fourth aspect of the disclosure provides a corresponding platelet counting method, including:
preparing a first test sample containing a first part of a test blood sample and a diluent, and preparing a second test sample containing a second part of the test blood sample, a hemolysis reagent for hemolyzing red blood cells and a first stain reagent;
allowing the first test sample to pass through a first flow chamber, and detecting electronic information as the first test sample passes through the first flow chamber;
allowing the second test sample to pass through a second flow chamber, irradiating the second test sample as the second test sample passes through the second flow chamber with a light, and detecting optical information generated by the second test sample after being irradiated with the light;
obtaining a second platelet counting result for the test blood sample based on the electronic information of the first test sample and the optical information of the second test sample; and
when the second platelet counting result is unreliable due to an abnormality of the test blood sample, preparing a third test sample containing a third part of the test blood sample, a diluent and a second stain reagent, allowing the third test sample to pass through the second flow chamber, irradiating the third test sample as the third test sample passes through the second flow chamber with a light, and detecting optical information generated by the third test sample after being irradiated with the light, so as to obtain and output a third platelet counting result for the test blood sample.

The platelet counting method according to the fourth aspect of the disclosure is particularly applicable to the sample analyzer according to the second aspect of the disclosure.

For the features and advantages of the sample analysis method according to the fourth aspect of the disclosure, reference can be made to the foregoing description of the sample analyzer according to the second aspect of the disclosure.

A fifth aspect of the disclosure further provides a platelet counting method for use in a hematology analyzer, the platelet counting method including:
determining whether a platelet optical measurement of the hematology analyzer is activated;
performing the platelet counting method provided in the third or fourth aspect of the disclosure when the platelet optical measurement of the hematology analyzer is determined to be not activated; and
when the platelet optical measurement of the hematology analyzer is determined to be activated, preparing a third test sample containing a third part of the test blood sample, a diluent and a second stain reagent, passing the third test sample through a second flow chamber, irradiating the third test sample passing through the second flow chamber with a light, and detecting optical information generated by the third test sample after being irradiated with the light, so as to obtain and output a third platelet counting result for the test blood sample.

In the platelet counting method provided in the fifth aspect of the disclosure, it is possible to determine whether to directly obtain and output the third platelet counting result for the test blood sample according to the determination of whether the platelet optical measurement of the hematology analyzer is activated.

### BRIEF DESCRIPTION OF THE DRAWINGS

FIG. 1 is a schematic structural diagram of an embodiment of a sample analyzer provided according to the disclosure;
FIG. 2 is a schematic structural diagram of an embodiment of an impedance testing device of the sample analyzer provided according to the disclosure;
FIG. 3 is a schematic structural diagram of an embodiment of an optical testing device of the sample analyzer according to the disclosure;
FIGS. 4 to 7 are schematic flowcharts of various embodiments of outputting a platelet counting result by means of a controller of the sample analyzer provided according to the disclosure;
FIG. 8 is a schematic flowchart of a first embodiment of determining an abnormal sample by means of the controller of the sample analyzer provided according to the disclosure;
FIG. 9 is a first platelet volume distribution histogram obtained by the controller of the sample analyzer provided according to the disclosure;
FIG. 10 is a schematic flowchart of a second embodiment of determining an abnormal sample by means of the controller of the sample analyzer provided according to the disclosure;
FIG. 11 is a second platelet volume distribution histogram obtained by the controller of the sample analyzer provided according to the disclosure; and
FIGS. 12 to 16 are schematic flowcharts of various embodiments of a platelet counting method provided according to the disclosure.

### DESCRIPTION OF THE PREFERRED EMBODIMENTS

The embodiments of the disclosure will be described below clearly and completely with reference to the accompanying drawings. Obviously, the embodiments described are merely some, rather than all, of the embodiments of the disclosure. Based on the embodiments of the disclosure, all the other embodiments which would have been obtained by those of ordinary skill in the art without any creative efforts shall fall within the scope of protection of the disclosure.

The inventors of the disclosure have recognized that during counting platelets by an impedance method, an abnormity of a blood sample may lead to an inaccurate platelet counting result for the blood sample. For example, if microcytes or red blood cell fragments are present in the blood sample, since the microcytes and the red blood cell fragments are similar in size to platelets, the microcytes and the red blood cell fragments may be erroneously recognized as the platelets during counting, resulting in a high platelet count. If large platelets are present in the blood sample, since the large platelets are similar in volume to red blood cells, the large platelets may be erroneously recognized as the red blood cells during counting, resulting in a low platelet count. If platelet aggregation is present in the blood sample, many platelets may aggregate into one cell, resulting in a low platelet count. All the above situations will lead to inaccurate platelet count, and in turn lead to incorrect clinical diagnosis.

In view of the limitation of platelet counting based on the impedance method, an optical platelet testing channel is usually added in the industry to count platelets, so as to eliminate the above interferences from an abnormal sample. However, adding an optical platelet testing channel requires additional clinical test costs, and the volume and complexity of the instrument will also increase significantly.

On this basis, the disclosure provides a technical solution of obtaining an accurate platelet counting result at a cost as low as possible.

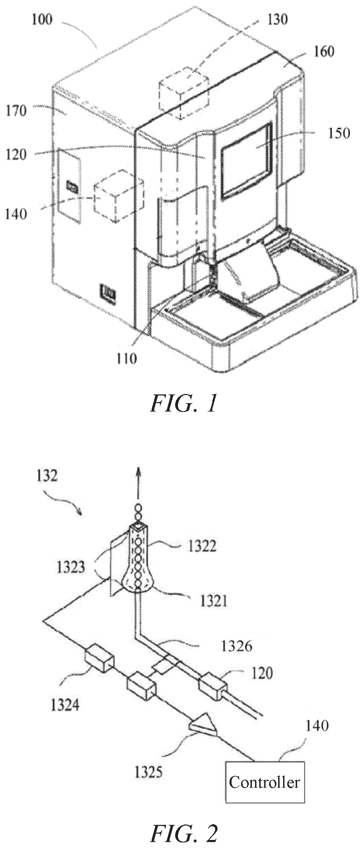

Referring to FIG. 1, the disclosure firstly provides a sample analyzer. The sample analyzer 100 includes at least a sample preparation device 120, a testing device 130, and a controller 140. In addition, the sample analyzer 100 may further include a sampling device 110 configured to collect a test blood sample from a patient.

The sampling device 110 may have a pipette (e.g., a sampling needle) with a pipette nozzle, and may have a drive portion that is configured to drive the pipette to quantitatively aspirate the test blood sample through the pipette nozzle. For example, the sampling needle is driven by the drive portion to move into a sample container holding the blood sample to aspirate the test blood sample.

The sample preparation device 120 is configured to prepare a first test sample containing a first part of the test blood sample and a diluent, and to prepare a second test sample containing a second part of the test blood sample, a hemolysis reagent for hemolyzing red blood cells, and a first stain reagent. Optionally, the sample preparation device 120 may be further configured to prepare a third test sample containing a third part of the test blood sample, a diluent and a second stain reagent.

In some embodiments, the sample preparation device 120 may have at least one reaction cell and a reagent supply device (not shown in the figures). The at least one reaction cell is configured to receive the test blood sample aspirated by the sampling device 110, and the reagent supply device supplies treatment reagents (including the diluent, the hemolysis reagent, the first stain reagent, the second stain reagent, etc.) to the at least one reaction cell, so that the test blood sample aspirated by the sampling device 110 is mixed, in the reaction cell, with the treatment reagents supplied by the reagent supply device, so as to prepare the test samples (including the first test sample and the second test sample).

In a specific example, the at least one reaction cell may include a first reaction cell and a second reaction cell, and the reagent supply device may include a first reagent supply portion and a second reagent supply portion. The sampling device 110 is configured to respectively dispense part of the aspirated test blood sample to the first reaction cell and the second reaction cell. The first reagent supply portion is configured to supply the diluent to the first reaction cell, so that a first part of the test blood sample that is dispensed to the first reaction cell is mixed and reacts with the diluent so as to prepare the first test sample. The second reagent supply portion is configured to supply the hemolysis reagent and the first stain reagent to the second reaction cell, so that a second part of the test blood sample that is dispensed to the second reaction cell is mixed and reacts with the hemolysis reagent and the first stain reagent so as to prepare the second test sample.

The hemolysis reagent herein is configured to lyse red blood cells in blood to break the red blood cells into fragments, with the morphology of white blood cells substantially unchanged. The first stain reagent may be a stain reagent configured to achieve leukocyte differential, for example, a stain reagent that can be used to achieve differential of leukocytes in a blood sample into at least three leukocyte subpopulations (monocytes, lymphocytes and neutrophils), or a stain reagent that can be used to recognize basophils and/or nucleated red blood cells in a blood sample.

In some embodiments, the hemolysis reagent may include at least one of alkyl glycosides, triterpenoid saponins and steroidal saponins, and the first stain reagent may include a membrane-specific dye or a mitochondria-specific dye. For more embodiments of the hemolysis reagent and the first stain reagent provided in the disclosure, reference can be made to PCT patent application WO 2019/206300 A1 submitted by the applicant and filed on 26 April 2019, which is incorporated herein by reference in its entirety.

Optionally, the at least one reaction cell may further include a third reaction cell, and the reagent supply device may further include a third reagent supply portion. The sample collection device 110 is further configured to respectively dispense part of the aspirated test blood sample to the third reaction cell. The third reagent supply portion is configured to supply the diluent and the second stain reagent to the third reaction cell, so that a third part of the test blood sample that is dispensed to the third reaction cell is mixed and reacts with the diluent and the second stain reagent so as to prepare a third test sample. The diluent herein is configured to spheroidize cells and has a staining promoting effect, and the second stain reagent is different from the first stain reagent and is a stain reagent that can be used to recognize platelets in a blood sample.

The testing device 130 includes an optical testing device 131 and an impedance testing device 132. The optical testing device 131 is configured to test the second test sample prepared by the sample preparation device 120 so as to obtain optical information of the second test sample (which is also referred to as a hemolysis optical testing channel), and is optionally configured to test the third test sample prepared by the sample preparation device 120 so as to obtain optical information of the third test sample (which is also referred to as an optical platelet testing channel). The impedance testing device 132 is configured to test the first test sample prepared by the sample preparation device 120 so as to obtain electronic information of the first test sample (which is also referred to as an impedance testing channel).

The impedance testing device 132 includes a first flow chamber and a detection component, the first flow chamber being configured to allow the first test sample to pass through, and the detection component being configured to obtain electronic information as the first test sample passes through the first flow chamber.

In an embodiment of the impedance testing device 132, the impedance testing device 132 is configured as a sheath flow impedance testing device. As shown in FIG. 2, the sheath flow impedance testing device 132 includes a first flow chamber 1321 having a hole 1322 with electrodes 1323. The sheath flow impedance testing device 132 measures DC impedance generated as particles in the first test sample pass through the hole 1322, and outputs electric signals (i.e., electronic information) reflecting information as the particles pass through the hole. Specifically, after aspirating the test blood sample, the sample collection device 110 is driven by a drive device thereof to move to the first reaction cell of the sample preparation device 120, and injects part of the aspirated test blood sample into the first reaction cell. The first test sample treated with the diluent in the first reaction cell is delivered to the first flow chamber 1321 through a delivery pipe 1326. The sheath flow impedance testing device 132 may be further provided with a sheath fluid compartment (not shown) configured to supply a sheath fluid to the first flow chamber 1321. In the first flow chamber 1321, the first test sample wrapped by the sheath fluid flows through the small hole 1322, so that the flow of the first test sample turns into a trickle flow, and thus the particles contained in the first test sample pass through the small hole 1322 one by one. The electrodes 1323 are electrically connected to a DC power source 1324, and the DC power source 1324 supplies DC power between the pair of electrodes 1323. When the DC power source 1324 supplies DC power, the impedance between the pair of electrodes 1323 can be detected, and resistance signals (i.e., the electronic information) representing impedance change are amplified by an amplifier 1325 and then transmitted to the controller 140. Since the magnitude of each resistance signal corresponds to the volume (size) of a particle, the resistance signals can be processed by the controller 140 to obtain a first platelet counting result.

The optical testing device 131 includes a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light as it passes through the second flow chamber. Optionally, the second flow chamber is further configured to allow the third test sample to pass through, the light source is further configured to irradiate the third test sample as the third test sample passes through the second flow chamber with light, and the optical detector is configured to detect the optical information generated by the third test sample after the third test sample is irradiated with the light as it passes through the second flow chamber.

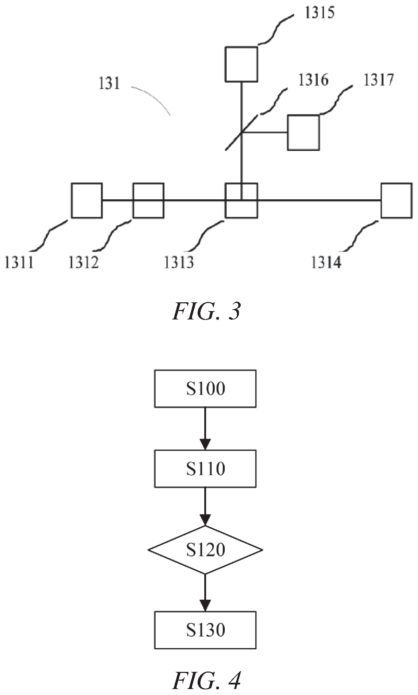

In an embodiment of the optical testing device 131, as shown in FIG. 3, the optical testing device 131 includes a light source 1311, a beam shaping assembly 1312, a second flow chamber 1313 and a forward-scattered light detector 1314, which are sequentially arranged in a straight line. On one side of the flow chamber 1313, a dichroscope 1316 is arranged at an angle of 45° to the straight line. Part of the lateral light emitted by blood cells in the flow chamber 1313 is transmitted through the dichroscope 1316 and captured by a fluorescence detector 1315 that is arranged behind the dichroscope 1316 and at an angle of 45° to the dichroscope 1316, and the other part of the lateral light is reflected by the dichroscope 1316 and captured by a side-scattered light detector 1317 arranged in front of the dichroscope 1316 and at an angle of 45° to the dichroscope 1316. Platelets in the blood sample can be counted according to optical information, such as forward-scattered light signals captured by the forward-scattered light detector 1314, side-scattered light signals captured by the side-scattered light detector 1317, and fluorescence signals captured by the fluorescence detector 1315.

The controller 140 includes a processor and a storage medium that stores a computer program. The controller 140 is configured to control, when the computer program is executed by the processor, the impedance testing device 132 and the optical testing device 131 to test the first test sample and the second test sample (and optionally the third test sample), so as to obtain and output a platelet counting result for the test blood sample.

In some embodiments, as shown in FIG. 1, the sample analyzer 100 may further include a display device 150, a first housing 160, and a second housing 170. The display device 150 is configured to display information associated with the platelet count, for example, display the platelet counting result. The testing device 130 and the controller 140 are arranged inside the second housing 170, and are respectively arranged on two sides of the second housing 170. The sample preparation device 120 is arranged inside the first housing 160. The display device 150 is arranged on an outer surface of the first housing 160.

Specific processes of obtaining and outputting the platelet counting result by means of the controller 140 of the sample analyzer provided according to the disclosure will be described below with reference to FIGS. 4 to 7.

In some embodiments, as shown in FIG. 4, the controller 140 may be configured to perform
step S100 of controlling the impedance testing device 132 to test the first test sample, so as to obtain a first platelet counting result PLT-I of the test blood sample based on the electronic information of the first test sample;
step S110 of controlling the optical testing device 131 to test the second test sample, so as to obtain a second platelet counting result PLT-W of the test blood sample based only on the optical information of the second test sample or obtain a second platelet counting result PLT-H of the test blood sample based on both the electronic information of the first test sample and the optical information of the second test sample;
step S120 of determining whether the first platelet counting result PLT-I is unreliable due to a first abnormality of the test blood sample; and
step S130 of outputting the first platelet counting result PLT-I and/or the second platelet counting result PLT-W/PLT-H according to the result of the determination.

It should be understood that step S100 and step S110 can be performed simultaneously or successively, which is not specifically limited in the disclosure.

It should be understood that in the disclosure, the second platelet counting result PLT-W or PLT-H is obtained using an existing hemolysis optical testing channel, such as a leukocyte differential counting channel or a nucleated red blood cell testing channel, rather than adding a special optical platelet testing channel as in the prior art. Since a blood routine test typically includes at least a platelet test based on an impedance method and a leukocyte differential, obtaining the first platelet counting result and the second platelet counting result basically has no increase in test cost.

In the disclosure, when the first platelet counting result PLT-I (i.e., the platelet counting result based on the impedance method) is determined to be reliable, the controller 140 may output the first platelet counting result PLT-I or the second platelet counting result PLT-W or PLT-H; and when the first platelet counting result is determined to be unreliable due to the first abnormality of the test blood sample, the controller 140 may further determine whether the second platelet counting result PLT-H is unreliable due to a second abnormality of the test blood sample or output the second platelet counting result PLT-W. Herein, the first abnormality and the second abnormality may be different from each other or may be the same.

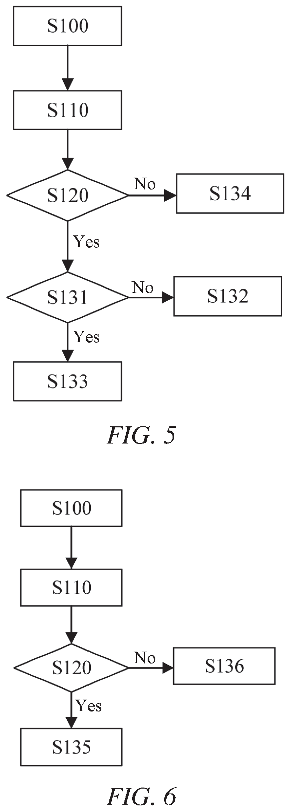

In some embodiments, as shown in FIG. 5, the controller 140 may be configured to, in step S110, obtain the second platelet counting result PLT-H of the test blood sample based on the electronic information of the first test sample and the optical information of the second test sample. For example, in an example, a first platelet histogram is generated from the electronic information of the first test sample, a second platelet histogram is generated from scattered light information in the optical information of the second test sample, and then the second platelet counting result PLT-H is calculated from the first platelet histogram and the second platelet histogram. For more embodiments and details, reference can be made to PCT patent application WO 2019/206313 A1 submitted by the applicant and filed on 26 April 2019, which is incorporated herein by reference in its entirety.

In this case, the controller 140 may be further configured to, in step S130, perform
step S131 of determining, when the first platelet counting result PLT-I is determined to be unreliable due to the first abnormality of the test blood sample, whether the second platelet counting result PLT-H is unreliable due to a second abnormality of the test blood sample;
step S132 of outputting the second platelet counting result PLT-H when the second platelet counting result PLT-H is determined to be reliable; and
step S133 of controlling, when the second platelet counting result PLT-H is determined to be unreliable due to the second abnormality of the test blood sample, the sample preparation device 120 to prepare the third test sample containing a third part of the test blood sample, the diluent and the second stain reagent, and controlling the optical testing device 131 to test the third test sample, so as to obtain and output a third platelet counting result PLT-O based on optical information of the third test sample.

Further, as shown in FIG. 5, the controller 140 may be further configured to, in step S130, perform step S134 of outputting the first platelet counting result PLT-I and/or the second platelet counting result PLT-H when the first platelet counting result PLT-I is determined to be reliable.

Studies have shown that the first platelet counting result PLT-I is unreliable when an interference from abnormal particles, such as microcytes, large-volume fragments (fragments with a volume greater than a predetermined value), large platelets, minimal-volume fragments (fragments with a volume less than a predetermined value) or platelet aggregation, etc., is present in the test blood sample. Furthermore, PLT-H is calculated from the electronic information of the first test sample and the optical information of the second test sample, and is thus similarly affected by the interference from abnormal particles. Particularly, when the platelet aggregation and/or at least a predetermined quantity of minimal-volume fragments is present in the test blood sample, PLT-H is unreliable. However, the third platelet counting result PLT-O is substantially not affected by the interference from abnormal particles. Thus, in the case where the first platelet counting result PLT-I of the test blood sample is unreliable, based on the further determination of whether the second platelet counting result PLT-H is reliable, it is possible to directly output the second platelet counting result PLT-H or trigger the test for the third platelet counting result PLT-O, thereby ensuring the accuracy of the platelet counting result for the abnormal sample.

In some other embodiments as an alternative to the embodiment shown in FIG. 5, as shown in FIG. 6, the controller 140 may be configured to, in step S110, obtain the second platelet counting result PLT-W of the test blood sample based only on the optical information of the second test sample, in a manner as described in PCT patent application WO 2019/206300 A1 submitted by the applicant and filed on 26 April 2019, which is incorporated herein by reference in its entirety.

In this case, the controller 140 may be further configured to, in step S130, perform step S135 of outputting the second platelet counting result PLT-W when the first platelet counting result PLT-I is determined to be unreliable due to the first abnormality of the test blood sample.

Further, as shown in FIG. 5, the controller 140 may be further configured to, in step S130, perform step S136 of outputting the first platelet counting result PLT-I and/or the second platelet counting result PLT-W when the first platelet counting result PLT-I is determined to be reliable.

Studies have shown that the first platelet counting result PLT-I is unreliable when an interference from particles, such as microcytes, large-volume fragments, large platelets, minimal-volume fragments or platelet aggregation, etc., is present in the test blood sample, but the second platelet counting result PLT-W obtained based only on the optical information of the second test sample containing the test blood sample will not be affected by these abnormal interferences. Thus, in the case where PLT-I is unreliable, PLT-W may be outputted directly; and in the case where PLT-I is reliable, either or both of PLT-I and PLT-W may be outputted, thereby ensuring the accuracy of the platelet counting result for the abnormal sample.

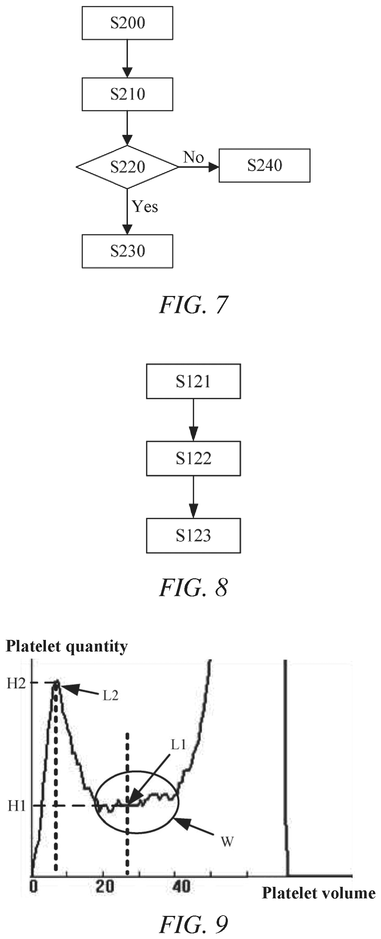

In some other embodiments as an alternative to the embodiment shown in FIG. 4, as shown in FIG. 7, the controller 140 may be configured to perform
step S200 of controlling the impedance testing device 132 to test the first test sample, so as to obtain the electronic information of the first test sample;
step S210 of controlling the optical testing device 131 to test the second test sample, so as to obtain the optical information of the second test sample;
step S220 of obtaining a second platelet counting result PLT-H of the test blood sample based on the electronic information of the first test sample and the optical information of the second test sample; and
step S230 of controlling, when the second platelet counting result PLT-H is unreliable due to an abnormality of the test blood sample, the sample preparation device 120 to prepare a third test sample containing a third part of the test blood sample, a diluent and a second stain reagent, and controlling the optical testing device 131 to test the third test sample, so as to obtain and output a third platelet counting result PLT-O based on optical information of the third test sample.

PLT-H is calculated from the electronic information of the first test sample and the optical information of the second test sample, and is thus affected by interferences from abnormal particles. Particularly, when platelet aggregation and/or at least a predetermined quantity of minimal-volume fragments is present in the test blood sample, PLT-H is unreliable. However, the third platelet counting result PLT-O is substantially not affected by interferences from abnormal particles. Thus, in the case where the second platelet counting result PLT-H of the test blood sample is unreliable, the test for the third platelet counting result PLT-O is triggered, thereby ensuring the accuracy of the platelet counting result for the abnormal sample.

Further, in the embodiment shown in FIG. 7, the controller 140 may be further configured to perform:
step S240 of outputting the second platelet counting result PLT-H when the second platelet counting result PLT-H is reliable.

As described above, the sample abnormality that leads to the first platelet counting result PLT-I being unreliable may include at least one of: presence of microcytes, large-volume fragments, large platelets, minimal-volume fragments and platelet aggregation in the test blood sample. The sample abnormality that leads to the second platelet counting result PLT-H, which is obtained based on the electronic information of the first test sample and the optical information of the second test sample, being unreliable may include at least one of: presence of platelet aggregation in the test blood sample, and presence of at least a predetermined quantity of fragments in the test blood sample that have a volume less than a predetermined value.

Specific processes of determining whether a sample abnormality is present in the test blood sample by means of the controller 140 will be described below with reference to FIGS. 8 to 11.

Studies have shown that microcytes, red blood cell fragments (including large-volume fragments and minimal-volume fragments), large platelets and platelet aggregation may lead to an abnormality in the platelet volume distribution histogram obtained based on the impedance method, especially lead to an abnormality of particle information distribution in a specific region of the platelet volume distribution histogram.

On this basis, in some embodiments, as shown in FIG. 8, the controller 140 may be further configured to, in step S120, perform:
step S121 of obtaining a first platelet volume distribution histogram based on the electronic information of the first test sample;
step S122 of determining whether particle information distribution in a specific region of the first platelet volume distribution histogram is abnormal; and
step S123 of determining, when the particle information distribution in the specific region is determined to be abnormal, that a sample abnormality which leads to the first platelet counting result PLT-I being unreliable is present in the test blood sample.

For example, the controller 140 may be further configured to perform the steps of:
obtaining a first boundary position of the first platelet volume distribution histogram for distinguishing a red blood cell volume distribution region from a platelet volume distribution region, wherein the specific region of the first platelet volume distribution histogram is located near the first boundary position;
obtaining a peak position of the platelet volume distribution region of the first platelet volume distribution histogram;
calculating a ratio of particle quantity corresponding to the first boundary position to particle quantity corresponding to the peak position in the first platelet volume distribution histogram; and
determining, based on the ratio, whether the particle information distribution in the specific region of the first platelet volume distribution histogram is abnormal.

FIG. 9 shows a first platelet volume distribution histogram obtained by the controller 140, wherein the abscissa represents the platelet volume, and the ordinate represents the platelet quantity.

As shown in FIG. 9, the specific region W of the first platelet volume histogram is located near the first boundary position L1, and the peak position of the platelet volume distribution region in the first platelet volume distribution histogram is represented by L2. The platelet quantity corresponding to the first boundary position L1 is represented by H1, and the platelet quantity corresponding to the peak position L2 is represented by H2.

In a specific example, if the ratio H1/H2 > M, where M represents a preset threshold, which may be, for example, a fixed constant, it can be determined that the particle information distribution in the specific region W is abnormal, and thus it can be determined that a sample abnormality which leads to PLT-I being unreliable is present in the test blood sample; and if the ratio H1/H2 ≤ M, it can be determined that the particle information distribution in the specific region W is normal, and thus it can be determined that no sample abnormality which leads to the PLT-I being unreliable is present in the test blood sample.

Alternatively or additionally, in some other embodiments, the controller 140 may be further configured to, in step S120, perform the steps of:
obtaining a mean corpuscular volume of the test blood sample based on the electronic information of the first test sample; and
determining, when the mean corpuscular volume is lower than a preset threshold, that a sample abnormality which leads to the first platelet counting result PLT-I being unreliable is present in the test blood sample.

For example, the preset threshold may be 70 fL, and when the mean corpuscular volume obtained based on the electronic information of the first test sample is lower than 70 fL, it is indicated that many microcytes are present in the test blood sample, which may result in falsely high PLT-I of the test blood sample, that is, it is indicated that a sample abnormality that leads to PLT-I being unreliable is present in the test blood sample.

Alternatively or additionally, in yet some other embodiments, the controller 140 may be further configured to determine whether platelet aggregation is present in the test blood sample based on the optical information of the second test sample, so as to determine whether a sample abnormality that leads to the first platelet counting result PLT-I being unreliable is present in the test blood sample.

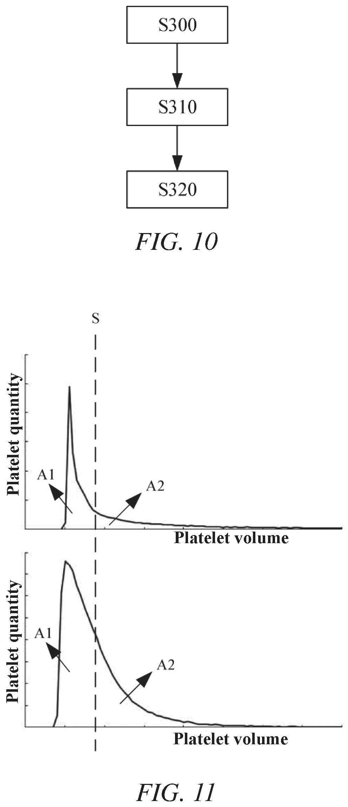

In some embodiments, as shown in FIG. 10, when determining whether the second platelet counting result PLT-H is unreliable due to the abnormality of the test blood sample, the controller 140 may be configured to perform:
step S300 of obtaining a second platelet volume distribution histogram based on the electronic information of the first test sample and the optical information of the second test sample;
step S310 of determining whether particle information distribution in a specific region of the second platelet volume distribution histogram is abnormal; and
step S320 of determining, when the particle information distribution in the specific region is determined to be abnormal, that a sample abnormality which leads to the second platelet counting result PLT-H being unreliable is present in the test blood sample.

Studies have shown that when an interference from minimal-volume fragments or platelet aggregation, etc., is present in the blood sample, the particle information distribution in the specific region in the second platelet volume distribution histogram is abnormal. Thus, it is possible to determine whether the particle information distribution in the specific region in the second platelet volume distribution histogram is abnormal so as to determine whether a sample abnormality that leads to PLT-H being unreliable is present in the test blood sample.

In a specific example, the controller 140 may be further configured to perform the steps of:
obtaining a second boundary, which corresponds to a preset platelet volume, in the second platelet volume distribution histogram, wherein the specific region of the second platelet volume distribution histogram is a platelet volume distribution region on the left of the second boundary;
calculating a first area on the left of the second boundary and a second area on the right of the second boundary below a curve of the second platelet volume distribution histogram; and
determining, according to the first area and the second area, whether the particle information distribution in the specific region of the second platelet volume distribution histogram is abnormal.

For example, if a ratio of the first area to the sum of the first area and the second area is greater than a preset threshold, or a ratio of the second area to the sum of the first area and the second area is less than the preset threshold, it can be determined that the particle information distribution in the specific region of the second platelet volume distribution histogram is abnormal, and thus it can be determined that a sample abnormality that leads to PLT-H being unreliable is present in the test blood sample.

FIG. 11 shows the second platelet volume distribution histograms of an abnormal blood sample and a normal blood sample, wherein the second platelet volume distribution histogram of the abnormal blood sample is shown on the upper side, and the second platelet volume distribution histogram of the normal blood sample is shown on the lower side, and wherein the abscissa represents the platelet volume, and the ordinate represents the platelet quantity.

When PLT-H is interfered, there will be significantly more particles in a small-volume region, and thus particle distribution information in the small-volume region will be abnormal. Thus, it can be determined whether PLT-H is interfered based on the abnormal distribution in the small-volume region.

As shown in FIG. 11, the second boundary of the second platelet volume distribution histogram is represented by S, and the specific region of the second platelet volume distribution histogram is a platelet volume distribution region on the left of the second boundary S, wherein the second boundary S may be fixed or floating. Below the curve of the second platelet volume distribution histogram, the first area on the left of the second boundary S is represented by A1, and the second area on the right of the second boundary S is represented by A2.

Assuming that N is a preset threshold, if A1/(A1 + A2) > N or A2/(A1 + A2) < N, it can be determined that the particle information distribution in the specific region of the second platelet volume distribution histogram is abnormal, and thus it can be determined that a sample abnormality that leads to PLT-H being unreliable is present in the corresponding test blood sample.

Alternatively, in some other embodiments, the controller 140 may be further configured to, when determining whether the second platelet counting result PLT-H is unreliable due to the abnormality of the test blood sample, perform the steps of determining whether platelet aggregation is present in the test blood sample based on the optical information of the second test sample.

In the embodiments of the disclosure, the second platelet counting result PLT-H or PLT-W is calculated from the optical information of the second test sample after being subjected to hemolysis and staining. Different from obtaining an optical platelet count by means of adding a special optical platelet testing channel as in the prior art, the second platelet counting result may be obtained using an existing hemolysis optical platelet testing channel in the disclosure.

On this basis, in some embodiments, the controller 140 may be further configured to obtain a leukocyte differential result and/or a leukocyte counting result and/or an immature granulocyte test result for the test blood sample based on the optical information of the second test sample. For example, in an example, the controller 140 may classify leukocytes into at least neutrophils, lymphocytes and monocytes based on the optical information of the second test sample.

Alternatively, in some other embodiments, the controller 140 may be further configured to recognize basophilic granulocyte and/or nucleated red blood cells in the test blood sample based on the optical information of the second test sample.

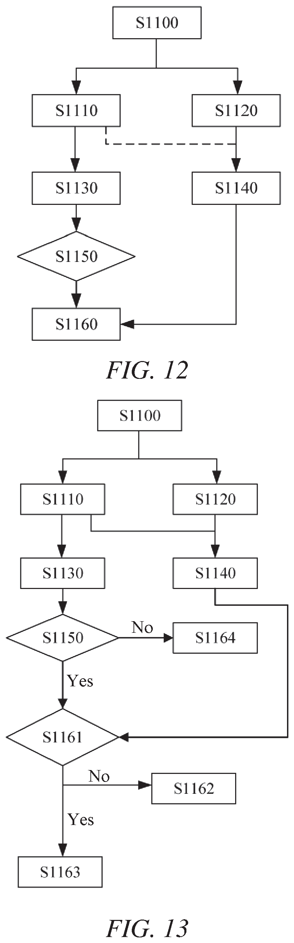

The disclosure further provides a platelet counting method. As shown in FIG. 12, the platelet counting method includes:
step S1100 of preparing a first test sample containing a first part of a test blood sample and a diluent, and preparing a second test sample containing a second part of the test blood sample, a hemolysis reagent for hemolyzing red blood cells and a first stain reagent;
step S1110 of allowing the first test sample to pass through a first flow chamber, and detecting electronic information as the first test sample passes through the first flow chamber;
step S1120 of allowing the second test sample to pass through a second flow chamber, irradiating the second test sample as the second test sample passes through the second flow chamber with a light, and detecting optical information generated by the second test sample after being irradiated with the light;
step S1130 of obtaining a first platelet counting result PLT-I for the test blood sample based on the electronic information of the first test sample;
step S1140 of obtaining a second platelet counting result PLT-W for the test blood sample based on only the optical information of the second test sample, or obtaining a second platelet counting result PLT-H for the test blood sample based on both the electronic information of the first test sample and the optical information of the second test sample;
step S1150 of determining whether the first platelet counting result PLT-I is unreliable due to a first abnormality of the test blood sample; and
step S1160 of outputting the first platelet counting result PLT-I and/or the second platelet counting result PLT-W/PLT-H according to the result of the determination.

In some embodiments, as shown in FIG. 13, step S1140 may include: obtaining the second platelet counting result PLT-H for the test blood sample based on the electronic information and the optical information. Accordingly, step S1160 includes:
step S1161 of determining, when the first platelet counting result PLT-I is determined to be unreliable due to the first abnormality of the test blood sample, whether the second platelet counting result PLT-H is unreliable due to a second abnormality of the test blood sample;
step S1162 of outputting the second platelet counting result PLT-H when the second platelet counting result PLT-H is determined to be reliable; and
step S1163 of preparing, when the second platelet counting result PLT-H is determined to be unreliable due to the second abnormality of the test blood sample, a third test sample containing a third part of the test blood sample, a diluent and a second stain reagent, allowing the third test sample to pass through the second flow chamber, irradiating the third test sample as the third test sample passes through the second flow chamber with a light, and detecting the optical information generated by the third test sample after being irradiated with the light, so as to obtain and output a third platelet counting result PLT-O for the test blood sample.

Further, in the embodiment shown in FIG. 13, step S1160 may further include step S1164 of outputting the first platelet counting result PLT-I and/or the second platelet counting result PLT-H when the first platelet counting result PLT-I is determined to be reliable.

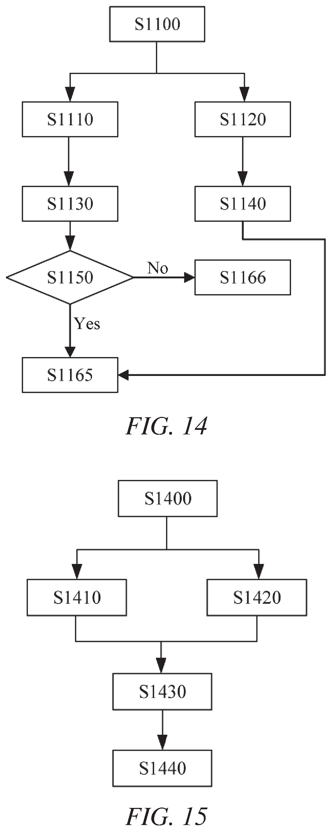

In some alternative embodiments, as shown in FIG. 14, step S1140 may include: obtaining the second platelet counting result PLT-W of the test blood sample based only on the optical information. Accordingly, step S1160 includes step S1165 of outputting the second platelet counting result PLT-W when the first platelet counting result PLT-I is determined to be unreliable due to the first abnormality of the test blood sample.

Further, in the embodiment shown in FIG. 14, step S1160 may further include step S1166 of outputting the first platelet counting result PLT-I and/or the second platelet counting result PLT-W when the first platelet counting result PLT-I is determined to be reliable.

In some embodiments, the platelet counting method may further include: obtaining a leukocyte differential result and/or a leukocyte counting result and/or an immature granulocyte test result for the test blood sample based on the optical information of the second test sample.

The disclosure further provides another platelet counting method. As shown in FIG. 15, the platelet counting method includes:
step S1400 of preparing a first test sample containing a first part of a test blood sample and a diluent, and preparing a second test sample containing a second part of the test blood sample, a hemolysis reagent for hemolyzing red blood cells, and a first stain reagent;
step S1410 of allowing the first test sample to pass through a first flow chamber, and detecting electronic information as the first test sample passes through the first flow chamber;
step S1420 of allowing the second test sample to pass through a second flow chamber, irradiating the second test sample as the second test sample passes through the second flow chamber with a light, and detecting optical information generated by the second test sample after being irradiated with the light;
step S1430 of obtaining the second platelet counting result PLT-H of the test blood sample based on the electronic information of the first test sample and the optical information of the second test sample; and
step S1440 of preparing, when the second platelet counting result PLT-H is unreliable due to the abnormality of the test blood sample, a third test sample containing a third part of the test blood sample, a diluent and a second stain reagent, allowing the third test sample to pass through the second flow chamber, light irradiating the third test sample as the third test sample passes through the second flow chamber with a light, and detecting optical information generated by the third test sample after being irradiated with the light, so as to obtain and output a third platelet counting result PLT-O of the test blood sample.

In some embodiments, the platelet counting method may further include: obtain a leukocyte differential result and/or a leukocyte counting result and/or an immature granulocyte test result for the test blood sample based on the optical information of the second test sample.

The platelet counting methods provided in the disclosure are particularly applicable to the sample analyzers provided in the disclosure. For the advantages and more embodiments of the platelet counting methods provided in the disclosure, reference can be made to the forgoing description of the sample analyzers, which will not be repeated herein.

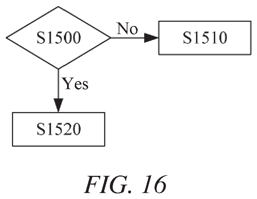

Another aspect of the disclosure further provides a platelet counting method for use in a hematology analyzer. As shown in FIG. 16, the platelet counting method includes:
step S1500 of determining whether a platelet optical measurement of the hematology analyzer is activated;
step S1510 of performing any one of the foregoing platelet counting methods provided in the disclosure when the platelet optical measurement of the hematology analyzer is determined to be not activated; and
step S1520 of preparing, when the platelet optical measurement of the hematology analyzer is determined to be activated, a third test sample containing a third part of the test blood sample, a diluent and a second stain reagent, allowing the third test sample to pass through a second flow chamber, irradiating the third test sample as the third test sample passes through the second flow chamber with a light, and detecting optical information generated by the third test sample after being irradiated with the light, so as to obtain and output a third platelet counting result PLT-O of the test blood sample.

The platelet optical measurement herein is a non-hemolysis optical test channel separately and specifically configured to carry out a platelet test in the hematology analyzer. When the hematology analyzer is provided with the non-hemolysis optical test channel, if a predetermined testing mode of the test blood sample originally includes a platelet optical measurement, the third platelet counting result obtained based on the platelet optical measurement is directly outputted, and the platelet counting method mentioned above is performed only when the predetermined testing mode of the test blood sample does not include the platelet optical measurement.

It should be noted that the term "first/second/third" in the embodiments of the disclosure is only used to distinguish similar objects, and does not represent specific order for the objects. It may be understood that "first/second/third" may be interchanged for specific order or chronological order when allowed.

The features or combinations thereof mentioned above in the description, accompanying drawings, and claims can be combined with each other arbitrarily or used separately as long as they are meaningful within the scope of the disclosure and do not contradict each other. The advantages and features described for the sample analyzers provided in the disclosure are applicable in a corresponding manner to the platelet counting methods provided in the disclosure, and vice versa. The foregoing description merely relates to the preferred embodiments of the disclosure, and is not intended to limit the scope of patent of the disclosure. All equivalent variations made by using the content of the specification and the accompanying drawings of the disclosure from the concept of the disclosure, or the direct/indirect applications of the contents in other related technical fields all fall within the scope of patent protection of the disclosure.

### Examples

1. A sample analyzer, comprising:
   a sample preparation device configured to prepare a first test sample containing a first part of a test blood sample and a diluent, and to prepare a second test sample containing a second part of the test blood sample, a hemolysis reagent for hemolyzing red blood cells and a first stain reagent;
   an impedance testing device comprising a first flow chamber and a detection component, the first flow chamber being configured to allow the first test sample to pass through, and the detection component being configured to obtain electronic information as the first test sample passes through the first flow chamber;
   an optical testing device comprising a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light as it passes through the second flow chamber; and
   a controller configured to:
      control the impedance testing device to test the first test sample, so as to obtain a first platelet counting result for the test blood sample based on the electronic information of the first test sample,
      control the optical testing device to test the second test sample, so as to obtain a second platelet counting result for the test blood sample based only on the optical information of the second test sample, or to obtain a second platelet counting result for the test blood sample based on both the electronic information of the first test sample and the optical information of the second test sample,
      determine whether the first platelet counting result is unreliable due to a first abnormality of the test blood sample, and
      output the first platelet counting result and/or the second platelet counting result according to the result of the determination.
2. The sample analyzer of example 1, wherein the controller is further configured to:
   obtain the second platelet counting result for the test blood sample based on the electronic information and the optical information;
   when the first platelet counting result is determined to be unreliable due to the first abnormality of the test blood sample, determine whether the second platelet counting result is unreliable due to a second abnormality of the test blood sample;
   output the second platelet counting result when the second platelet counting result is determined to be reliable; and
   when the second platelet counting result is determined to be unreliable due to the second abnormality of the test blood sample, control the sample preparation device to prepare a third test sample containing a third part of the test blood sample, a diluent and a second stain reagent, and control the optical testing device to test the third test sample so as to obtain and output a third platelet counting result for the test blood sample based on optical information of the third test sample.
3. The sample analyzer of example 1, wherein the controller is further configured to:
   obtain the second platelet counting result for the test blood sample based only on the optical information; and
   output the second platelet counting result when the first platelet counting result is determined to be unreliable due to the first abnormality of the test blood sample.
4. The sample analyzer of any one of examples 1 to 3, wherein the controller is further configured to:
   output the first platelet counting result and/or the second platelet counting result when the first platelet counting result is determined to be reliable.
5. The sample analyzer of example 2, wherein the second abnormality that leads to the second platelet counting result being unreliable comprises at least one of:
   presence of platelet aggregation in the test blood sample; and
   presence of at least a predetermined quantity of fragments in the test blood sample that have a volume less than a predetermined value.
6. The sample analyzer of any one of examples 1 to 5, wherein the controller is further configured to:
   obtain a first platelet volume distribution histogram based on the electronic information of the first test sample;
   determine whether particle information distribution in a specific region of the first platelet volume distribution histogram is abnormal; and
   when the particle information distribution in the specific region is determined to be abnormal, determine that a sample abnormality that leads to the first platelet counting result being unreliable is present in the test blood sample.
7. The sample analyzer of example 6, wherein the controller is further configured to:
   obtain a first boundary position in the first platelet volume distribution histogram for distinguishing a red blood cell volume distribution region from a platelet volume distribution region, the specific region of the first platelet volume distribution histogram being located near the boundary position;
   obtain a peak position in the platelet volume distribution region of the first platelet volume distribution histogram;
   calculate a ratio of particle quantity corresponding to the first boundary position to particle quantity corresponding to the peak position in the first platelet volume distribution histogram; and
   determine, based on the ratio, whether the particle information distribution in the specific region of the first platelet volume distribution histogram is abnormal.
8. The sample analyzer of any one of examples 1 to 7, wherein the controller is further configured to:
   obtain a mean corpuscular volume of the test blood sample based on the electronic information of the first test sample; and
   when the mean corpuscular volume is lower than a preset threshold, determine that a sample abnormality which leads to the first platelet counting result being unreliable is present in the test blood sample.
9. The sample analyzer of example 2 or 5, wherein the controller is further configured to:
   obtain a second platelet volume distribution histogram based on the electronic information of the first test sample and the optical information of the second test sample;
   determine whether particle information distribution in a specific region of the second platelet volume distribution histogram is abnormal; and
   when the particle information distribution in the specific region is determined to be abnormal, determine that a sample abnormality which leads to the second platelet counting result being unreliable is present in the test blood sample.
10. The sample analyzer of example 9, wherein the controller is further configured to:
   obtain a second boundary, which corresponds to a preset platelet volume, in the second platelet volume distribution histogram, the specific region of the second platelet volume distribution histogram being a platelet volume distribution region on the left of the second boundary;
   calculate a first area on the left of the second boundary and a second area on the right of the second boundary below a curve of the second platelet volume distribution histogram; and
   determine, according to the first area and the second area, whether the particle information distribution in the specific region of the second platelet volume distribution histogram is abnormal.
11. The sample analyzer of any one of examples 1 to 10, wherein the controller is further configured to determine, based on the optical information of the second test sample, whether platelet aggregation is present in the test blood sample, so as to determine whether a sample abnormality of the test blood sample that leads to the first platelet counting result or the second platelet counting result being unreliable is present in the test blood sample.
12. The sample analyzer of any one of examples 1 to 11, wherein the controller is further configured to:
   obtain a leukocyte differential result and/or a leukocyte counting result and/or an immature granulocyte test result for the test blood sample based on the optical information of the second test sample.
13. A sample analyzer, comprising:
   a sample preparation device configured to prepare a first test sample containing a first part of a test blood sample and a diluent, and to prepare a second test sample containing a second part of the test blood sample, a hemolysis reagent for hemolyzing red blood cells and a first stain reagent;
   an impedance testing device comprising a first flow chamber and a detection component, the first flow chamber being configured to allow the first test sample to pass through, and the detection component being configured to obtain electronic information as the first test sample passes through the first flow chamber;
   an optical testing device comprising a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light as it passes through the second flow chamber; and
   a controller configured to:
      control the impedance testing device to test the first test sample, so as to obtain the electronic information of the first test sample,
      control the optical testing device to test the second test sample, so as to obtain the optical information of the second test sample,
      obtain a second platelet counting result for the test blood sample based on the electronic information of the first test sample and the optical information of the second test sample, and
      when the second platelet counting result is unreliable due to an abnormality of the test blood sample, control the sample preparation device to prepare a third test sample containing a third part of the test blood sample, a diluent and a second stain reagent, and control the optical testing device to test the third test sample, so as to obtain and output a third platelet counting result based on optical information of the third test sample.
14. The sample analyzer of example 13, wherein
   the sample abnormality that leads to the second platelet counting result being unreliable comprises at least one of:
   presence of platelet aggregation in the test blood sample; and
   presence of at least a predetermined quantity of fragments in the test blood sample that have a volume less than a predetermined value.
15. The sample analyzer of example 13 or 14, wherein the controller is further configured to:
   obtain a second platelet volume distribution histogram based on the electronic information of the first test sample and the optical information of the second test sample;
   determine whether the particle information distribution in a specific region of the second platelet volume distribution histogram is abnormal; and
   when the particle information distribution in the specific region is determined to be abnormal, determine that a sample abnormality which leads to the second platelet counting result being unreliable is present in the test blood sample.
16. The sample analyzer of example 15, wherein the controller is further configured to:
   obtain a second boundary, which corresponds to a preset platelet volume, in the second platelet volume distribution histogram, the specific region of the second platelet volume distribution histogram being a platelet volume distribution region on the left of the second boundary;
   calculate a first area on the left of the second boundary and a second area on the right of the second boundary below a curve of the second platelet volume distribution histogram; and
   determine, according to the first area and the second area, whether the particle information distribution in the specific region of the second platelet volume distribution histogram is abnormal.
17. The sample analyzer of example 14, wherein the controller is further configured to determine, based on the optical information of the second test sample, whether platelet aggregation is present in the test blood sample.
18. The sample analyzer of any one of examples 13 to 17, wherein the controller is further configured to:
   obtain a leukocyte differential result and/or a leukocyte counting result and/or an immature granulocyte test result for the test blood sample based on the optical information of the second test sample.
19. A platelet counting method, comprising:
   preparing a first test sample containing a first part of a test blood sample and a diluent, and preparing a second test sample containing a second part of the test blood sample, a hemolysis reagent for hemolyzing red blood cells and a first stain reagent;
   allowing the first test sample to pass through a first flow chamber, and detecting electronic information as the first test sample passes through the first flow chamber;
   allowing the second test sample to pass through a second flow chamber, irradiating the second test sample as the second test sample passes through the second flow chamber with a light, and detecting optical information generated by the second test sample after being irradiated with the light;
   obtaining a first platelet counting result for the test blood sample based on the electronic information of the first test sample;
   obtaining a second platelet counting result for the test blood sample based only on the optical information of the second test sample, or obtaining a second platelet counting result for the test blood sample based on both the electronic information of the first test sample and the optical information of the second test sample;
   determining whether the first platelet counting result is unreliable due to a first abnormality of the test blood sample; and
   outputting the first platelet counting result and/or the second platelet counting result based on the result of the determination.
20. The method of example 19, further comprising:
   obtaining the second platelet counting result for the test blood sample based on the electronic information and the optical information;
   when the first platelet counting result is determined to be unreliable due to the first abnormality of the test blood sample, determining whether the second platelet counting result is unreliable due to a second abnormality of the test blood sample;
   outputting the second platelet counting result when the second platelet counting result is determined to be reliable; and
   when the second platelet counting result is determined to be unreliable due to the second abnormality of the test blood sample, preparing a third test sample containing a third part of the test blood sample, a diluent and a second stain reagent, passing the third test sample through the second flow chamber, irradiating the third test sample passing through the second flow chamber with a light, and detecting the optical information generated by the third test sample after being irradiated with the light, so as to obtain and output a third platelet counting result for the test blood sample.
21. The method of example 19, further comprising:
   obtaining the second platelet counting result for the test blood sample based only on the optical information; and
   outputting the second platelet counting result when the first platelet counting result is determined to be unreliable due to the first abnormality of the test blood sample.
22. The method of any one of examples 19 to 21, further comprising
   outputting the first platelet counting result and/or the second platelet counting result when the first platelet counting result is determined to be reliable.
23. A platelet counting method, comprising:
   preparing a first test sample containing a first part of a test blood sample and a diluent, and preparing a second test sample containing a second part of the test blood sample, a hemolysis reagent for hemolyzing red blood cells and a first stain reagent;
   allowing the first test sample to pass through a first flow chamber, and detecting electronic information as the first test sample passes through the first flow chamber;
   allowing the second test sample to pass through a second flow chamber, irradiating the second test sample as the second test sample passes through the second flow chamber with a light, and detecting optical information generated by the second test sample after being irradiated with the light;
   obtaining a second platelet counting result for the test blood sample based on the electronic information of the first test sample and the optical information of the second test sample; and
   when the second platelet counting result is unreliable due to an abnormality of the test blood sample, preparing a third test sample containing a third part of the test blood sample, a diluent and a second stain reagent, allowing the third test sample to pass through the second flow chamber, irradiating the third test sample passing through the second flow chamber with a light, and detecting the optical information generated by the third test sample after being irradiated with the light, so as to obtain and output a third platelet counting result for the test blood sample.
24. The method of any one of examples 19 to 23, further comprising:
   obtaining a leukocyte differential result and/or a leukocyte counting result and/or an immature granulocyte test result for the test blood sample based on the optical information of the second test sample.
25. A platelet counting method for use in a hematology analyzer, the method comprising:
   determining whether a platelet optical measurement of the hematology analyzer is activated;
   performing the platelet counting method of any one of examples 19 to 24 when the platelet optical measurement of the hematology analyzer is determined to be not activated; and
   when the platelet optical measurement of the hematology analyzer is determined to be activated, preparing a third test sample containing a third part of the test blood sample, a diluent and a second stain reagent, passing the third test sample through a second flow chamber, irradiating the third test sample passing through the second flow chamber with a light, and detecting optical information generated by the third test sample after being irradiated with the light, so as to obtain and output a third platelet counting result for the test blood sample.

## Claims

1. A sample analyzer, comprising:
a sample preparation device configured to prepare a first test sample containing a first part of a test blood sample and a diluent, and to prepare a second test sample containing a second part of the test blood sample, a hemolysis reagent for hemolyzing red blood cells and a first stain reagent;
an impedance testing device comprising a first flow chamber and a detection component, the first flow chamber being configured to allow the first test sample to pass through, and the detection component being configured to obtain electronic information as the first test sample passes through the first flow chamber;
an optical testing device comprising a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light as it passes through the second flow chamber; and
a controller configured to:
control the impedance testing device to test the first test sample, so as to obtain the electronic information of the first test sample,
control the optical testing device to test the second test sample, so as to obtain the optical information of the second test sample,
obtain a second platelet counting result for the test blood sample based on the electronic information of the first test sample and the optical information of the second test sample, and
when the second platelet counting result is unreliable due to an abnormality of the test blood sample, control the sample preparation device to prepare a third test sample containing a third part of the test blood sample, a diluent and a second stain reagent, and control the optical testing device to test the third test sample, so as to obtain and output a third platelet counting result based on optical information of the third test sample.

2. The sample analyzer of claim 1, wherein
the sample abnormality that leads to the second platelet counting result being unreliable comprises at least one of:
presence of platelet aggregation in the test blood sample; and
presence of at least a predetermined quantity of fragments in the test blood sample that have a volume less than a predetermined value.

3. The sample analyzer of claim 1 or 2, wherein the controller is further configured to:
obtain a second platelet volume distribution histogram based on the electronic information of the first test sample and the optical information of the second test sample;
determine whether the particle information distribution in a specific region of the second platelet volume distribution histogram is abnormal; and
when the particle information distribution in the specific region is determined to be abnormal, determine that a sample abnormality which leads to the second platelet counting result being unreliable is present in the test blood sample.

4. The sample analyzer of claim 3, wherein the controller is further configured to:
obtain a second boundary, which corresponds to a preset platelet volume, in the second platelet volume distribution histogram, the specific region of the second platelet volume distribution histogram being a platelet volume distribution region on the left of the second boundary;
calculate a first area on the left of the second boundary and a second area on the right of the second boundary below a curve of the second platelet volume distribution histogram; and
determine, according to the first area and the second area, whether the particle information distribution in the specific region of the second platelet volume distribution histogram is abnormal.

5. The sample analyzer of claim 2, wherein the controller is further configured to determine, based on the optical information of the second test sample, whether platelet aggregation is present in the test blood sample.

6. The sample analyzer of any one of claims 2 to 6, wherein the controller is further configured to:
obtain a leukocyte differential result and/or a leukocyte counting result and/or an immature granulocyte test result for the test blood sample based on the optical information of the second test sample.

7. A platelet counting method, comprising:
preparing a first test sample containing a first part of a test blood sample and a diluent, and preparing a second test sample containing a second part of the test blood sample, a hemolysis reagent for hemolyzing red blood cells and a first stain reagent;
allowing the first test sample to pass through a first flow chamber, and detecting electronic information as the first test sample passes through the first flow chamber;
allowing the second test sample to pass through a second flow chamber, irradiating the second test sample as the second test sample passes through the second flow chamber with a light, and detecting optical information generated by the second test sample after being irradiated with the light;
obtaining a second platelet counting result for the test blood sample based on the electronic information of the first test sample and the optical information of the second test sample; and
when the second platelet counting result is unreliable due to an abnormality of the test blood sample, preparing a third test sample containing a third part of the test blood sample, a diluent and a second stain reagent, allowing the third test sample to pass through the second flow chamber, irradiating the third test sample passing through the second flow chamber with a light, and detecting the optical information generated by the third test sample after being irradiated with the light, so as to obtain and output a third platelet counting result for the test blood sample.

8. The method of claim 7, further comprising:
obtaining a leukocyte differential result and/or a leukocyte counting result and/or an immature granulocyte test result for the test blood sample based on the optical information of the second test sample.

9. A platelet counting method for use in a hematology analyzer, the method comprising:
determining whether a platelet optical measurement of the hematology analyzer is activated;
performing the platelet counting method of any one of claims 1 to 6 when the platelet optical measurement of the hematology analyzer is determined to be not activated; and
when the platelet optical measurement of the hematology analyzer is determined to be activated, preparing a third test sample containing a third part of the test blood sample, a diluent and a second stain reagent, passing the third test sample through a second flow chamber, irradiating the third test sample passing through the second flow chamber with a light, and detecting optical information generated by the third test sample after being irradiated with the light, so as to obtain and output a third platelet counting result for the test blood sample.

10. A sample analyzer, comprising:
a sample preparation device configured to prepare a first test sample containing a first part of a test blood sample and a diluent, and to prepare a second test sample containing a second part of the test blood sample, a hemolysis reagent for hemolyzing red blood cells and a first stain reagent;
an impedance testing device comprising a first flow chamber and a detection component, the first flow chamber being configured to allow the first test sample to pass through, and the detection component being configured to obtain electronic information as the first test sample passes through the first flow chamber;
an optical testing device comprising a second flow chamber, a light source and an optical detector, the second flow chamber being configured to allow the second test sample to pass through, the light source being configured to irradiate the second test sample as the second test sample passes through the second flow chamber with a light, and the optical detector being configured to detect optical information generated by the second test sample after the second test sample is irradiated with the light as it passes through the second flow chamber; and
a controller configured to:
control the impedance testing device to test the first test sample, so as to obtain a first platelet counting result for the test blood sample based on the electronic information of the first test sample,
control the optical testing device to test the second test sample, so as to obtain a second platelet counting result for the test blood sample based on both the electronic information of the first test sample and the optical information of the second test sample,
determine whether the first platelet counting result is unreliable due to a first abnormality of the test blood sample, and
output the first platelet counting result and/or the second platelet counting result according to the result of the determination.

11. The sample analyzer of claim 11, wherein the controller is further configured to:
obtain a first platelet volume distribution histogram based on the electronic information of the first test sample;
determine whether particle information distribution in a specific region of the first platelet volume distribution histogram is abnormal; and
when the particle information distribution in the specific region is determined to be abnormal, determine that a sample abnormality that leads to the first platelet counting result being unreliable is present in the test blood sample.

12. The sample analyzer of claim 11, wherein the controller is further configured to:
obtain a first boundary position in the first platelet volume distribution histogram for distinguishing a red blood cell volume distribution region from a platelet volume distribution region, the specific region of the first platelet volume distribution histogram being located near the boundary position;
obtain a peak position in the platelet volume distribution region of the first platelet volume distribution histogram;
calculate a ratio of particle quantity corresponding to the first boundary position to particle quantity corresponding to the peak position in the first platelet volume distribution histogram; and
determine, based on the ratio, whether the particle information distribution in the specific region of the first platelet volume distribution histogram is abnormal.

13. The sample analyzer of any one of claims 10 to 12, wherein the controller is further configured to:
obtain a mean corpuscular volume of the test blood sample based on the electronic information of the first test sample; and
when the mean corpuscular volume is lower than a preset threshold, determine that a sample abnormality which leads to the first platelet counting result being unreliable is present in the test blood sample.

14. The sample analyzer of any one of claims 10 to 13, wherein the controller is further configured to:
obtain a leukocyte differential result and/or a leukocyte counting result and/or an immature granulocyte test result for the test blood sample based on the optical information of the second test sample.
